# EUROPEAN PATENT APPLICATION

(11) **EP 1 542 101 A1**
(43) Date of publication of application: **15.06.2005**
(21) Application number: 03797719.6
(22) Date of filing: 19.09.2003
(51) Int. Cl.: G05B 13/02

(54) **INTERACTIVE DEVICE**

(30) Priority: 20.09.2002 JP 2002276121
(71) Applicant: MATSUSHITA ELECTRIC INDUSTRIAL CO., LTD., Kadoma-shi, Osaka 571-8501 (JP)
(72) Inventor: YAMAMOTO, Shinichi, Yokohama-shi, Kanagawa 222-0011 (JP); YAMAMOTO, Hiroshi, Shijonawate-shi, Osaka 575-0013 (JP)
(74) Representative: Pautex Schneider, Nicole
(86) International application number: PCT/JP2003/012040
(87) International publication number: WO 2004/027527

(57) **Abstract**

An interactive apparatus **1** which is able to decide on an action pattern in accordance with health conditions of a user without a necessity of putting a biometric sensor on a human body is provided. The interactive apparatus **1** comprises: detection means **50b** for detecting a health condition of a user; deciding means **50c** for deciding on an action pattern in accordance with the health condition of the user; execution instructing means **50g** for instructing execution of the action pattern; offering means **50e** for making an offer of the action pattern to the user with a speech before instructing execution of the action pattern; and determination means **50f** for determining whether an answer of the user to the offered action pattern is an answer to accept the offered action pattern or not. The execution instructing means **50g** instructs execution of the offered action pattern when the answer of the user is determined to be the answer to accept the offered action pattern.

## Description

### TECHNICAL FIELD

The present invention relates to an interactive apparatus which can have a conversation with a user.

### BACKGROUND ART

An audio apparatus which monitors for behavior information for reproducing an audio signal of preference of a sequential habitant at a level adjusted in accordance with the current situation and the physical condition of the user (see, for example, Japanese Laid-Open Publication No. 11-221196). The audio apparatus detects the situation of the habitant by using a sensor provided in a room. The audio apparatus monitors identification information and behavior information from a portable transceiver (including a biometric sensor) worn by the habitant, and adjusts the audio signal of the preference of the sequential habitant to a level in accordance with the current situation and the physical condition of the habitant for reproduction.

However, in the conventional art as described in Japanese Laid-Open Publication No. 11-221196, the habitant has to wear a portable transceiver for acquisition of biometric information and the like. Wearing the sensor is cumbersome for the habitant, and thus, this method is inconvenient. There is also a problem that the habitant is monitored all the time by the sensor provided in the room, which may cause uncomfortable feeling of the habitant.

The object of the present invention is to provide an interactive apparatus which is able to decide on an action pattern in accordance with the health conditions of the user without a necessity of putting a biometric sensor on a human body.

### DISCLOSURE OF THE INVENTION

An interactive apparatus according to the present invention comprises: detection means for detecting a health condition of a user; deciding means for deciding on an action pattern in accordance with the health condition of the user detected by the detection moans; execution instructing means for instructing execution of the action pattern decided by the deciding means; offering means for making an offer of the action pattern to the user with a speech before instructing execution of the action pattern decided by the deciding means ; and determination means for determining whether an answer of the user to the offered action pattern is an answer to accept the offered action pattern or not, in which the execution instructing means instructs execution of the offered action pattern when the answer of the user is determined to be the answer to accept the offered action pattern, thereby achieving the above-described object.

The detection means may detect the health condition of the user based on utterance of the user.

The detection means may detect the health condition of the user based on keywords uttered by the user.

Offer necessity determination means for determining whether it is required to make an offer of the action pattern to the user before instructing execution of the action pattern decided by the deciding means may be further included, and the offering means may make an offer of the action pattern to the user with a speech when it is determined that making an offer of the action pattern to the user is required before instructing execution of the action pattern.

The offer necessity determination means may determine necessity of making an offer in accordance with a value of a flag indicating a necessity of making an offer which is previously allocated to the action pattern.

The offer necessity determination means may determine necessity of making an offer based on time distribution of the number of times the action pattern is performed.

The deciding means may decide one of a plurality of action patterns to which priorities are respectively allocated as an action pattern in accordance with the health condition of the user, and may change the priority allocated to the action pattern in accordance with whether or not the action pattern is accepted by the user.

Storage means for storing the action pattern in accordance with the health condition of the usermay be further included, and the deciding means may decide on the action pattern by using the action pattern stored in the storage means.

The action pattern offered by the offering means to the user may include selecting contents to be reproduced by a reproducing device.

The contents may include audio data, video data, and lighting control data, and the reproducing device may change at least one of light intensity and color of light of a lighting apparatus based on the lighting control data.

The interactive device may have at least one of an agent function and a traveling function.

The health condition of the user may represent at least one of feelings of the user and a physical condition of the user.

An interactive apparatus according to the present invention comprises: a voice input section for converting a voice produced by the user into a voice signal, a voice recognition section for recognizing words uttered by the user based on the voice signal output from the voice input section; a conversation database in which words expected to be uttered by the user are previously registered, and which stores correspondences between the registered words and the health condition of the user; detection means for detecting the health condition of the user by checking the words recognized by the voice recognition section against the words registered in the conversation database, and deciding on the health condition of the user in accordance with the checking result; deciding means for deciding on an action pattern in accordance with the health condition of the user detected by the detection means based on an action pattern table storing correspondences between the health condition of the user and action patterns of the interactive apparatus; execution instructing means for instructing execution of the action pattern decided by the deciding means; offering means for synthesizing an offering sentence based on an output result of the detection means and an output result of the deciding means and making an offer of the action pattern to the user with a speech before instructing execution of the action pattern decided by the deciding means: and determination means for determining whether an answer of the user to the offered action pattern is an answer to accept the offered action pattern or not, in which the execution instructing means instructs execution of the offered action pattern when the answer of the user is determined to be the answer to accept the offered action pattern, thereby achieving the above-described object.

Means for receiving an action pattern which is counter-offered by the user with respect to the offered action pattern, means for the interactive apparatus to determine whether the counter-offered action pattern is executable or not, and means for updating the correspondences between the health condition of the user and the action patterns of the interactive apparatus which are stored in the action pattern table when the interactive apparatus determines that the counter-offered action pattern is executable may be further included.

### BRIEF DESCRIPTION OF THE DRAWINGS

Figure **1** is a diagram showing an appearance of a robot **1** as an example of an interactive apparatus according to the present invention.
Figure **2** is a diagram showing an exemplary internal structure of the robot **1**.
Figure **3** is a diagram showing exemplary relationships between keywords to be generated by a user which are stored in a conversation database **140** and the health conditions of the user.
Figure **4** is a diagram showing exemplary relationships between the health conditions of the user which are stored in an information database **160** and an action pattern of the robot **1**.
Figure **5** is a flow chart showing an exemplary procedure for the robot **1** to detect the health condition of the user and indicate execution of an action pattern which matches the health condition of the user.
Figure **6** is a diagram showing an exemplary structure of a reproducing apparatus **2100** which allows synchronized reproduction of audio data and/or video data, and lighting control data.
Figure **7** is a diagram showing an exemplary internal structure of a voice recognition section **40.**
Figure **8a** is a diagram showing an exemplary internal structure of a processing section **50** shown in Figure **2.**
Figure **8b** is a diagram showing another exemplary internal structure of the processing section **50** shown in Figure **2.**
Figure **8c** is a diagram showing another exemplary internal structure of the processing section **50** shown in Figure **2.**
Figure **9** is a diagram for illustrating how offering means **50e** create offering sentences.
Figure **10** is a diagram showing an exemplary internal structure of offer necessity determination means **50d.**
Figure **11** is a diagram showing an exemplary structure of an action offer necessity table **162.**

### BEST MODE FOR CARRYING OUT THE INVENTION

Hereinafter, the embodiments of the present invention will be described with reference to the drawings.

As used herein, a "health condition of a user" refers to at least one of the feeling or a physical condition of a user. A "user" refers to an owner of the interactive apparatus.

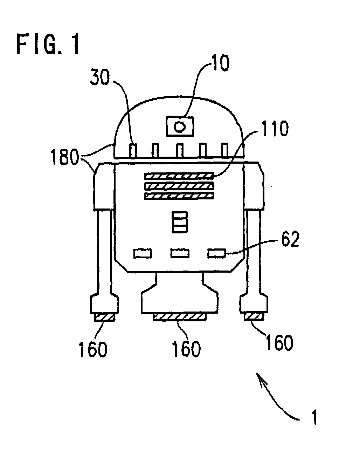

Figure **1** shows an appearance of a robot **1** as an example of an interactive apparatus according to the present invention. The robot **1** is formed such that it can have conversation with a user.

The robot **1** shown in Figure **1** includes: a camera **10** which corresponds to an "eye"; a speaker **110** and an antenna **62** which correspond to a "mouth"; a microphone **30** and an antenna **62** which correspond to an "ear"; and movable sections **180** which correspond to a "neck" and an "arm".

The robot **1** may be an autonomous traveling robot (a mobile robot) having traveling sections **160** which allows it to travel by itself, or may be of a type which cannot be moved by itself.

Any mechanism may be adopted as a mechanism for allowing the robot **1** to travel. For example, the robot **1** may be formed so as to move forward or backward by controlling rotations of rollers provided on hands and feet. Alternatively, the robot **1** may be a mobile robot using tires or legs. The robot **1** may be a human-shaped robot which imitates an animal walking upright with two legs such as human, or may be a pet robot which imitates an animal walking with four legs.

The interactive robot has been illustrated as an example of interactive apparatuses. However, the interactive apparatuses are not limited to this. The interactive apparatuses may be any apparatus formed such that it can have a conversation with users. The interactive apparatuses may be, for example, interactive toys, interactive portable devices (including mobile phones), or interactive agents.

It is preferable that the interactive agents have function of getting around an information space such as Internet, and performing information processing such as search for information, filtering, scheduling and the like on behalf of humans (software agent function). The interactive agents have conversation with humans as if they are humans. Thus, they may be sometimes called anthropomorphic agents.

The interactive apparatuses may have at least one of an agent function and a traveling function.

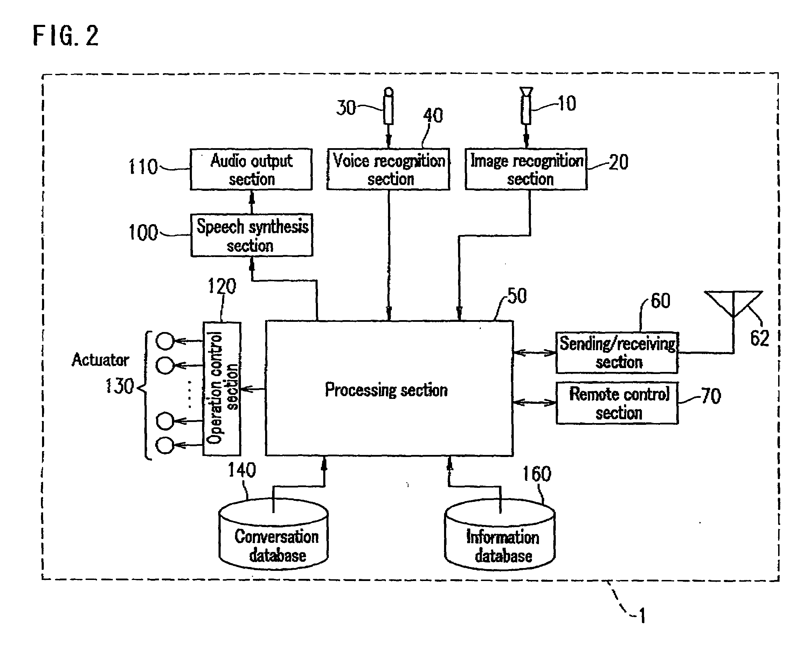

Figure **2** shows an exemplary internal structure of the robot **1.**

An image recognition section **20** captures image from a camera **10** (image input section), recognizes the captured image, and outputs the recognized result to a processing section **50.**

A voice recognition section **40** captures voice from a microphone **30** (voice input section), recognizes the captured voice, and outputs the recognized result to the processing section **50.**

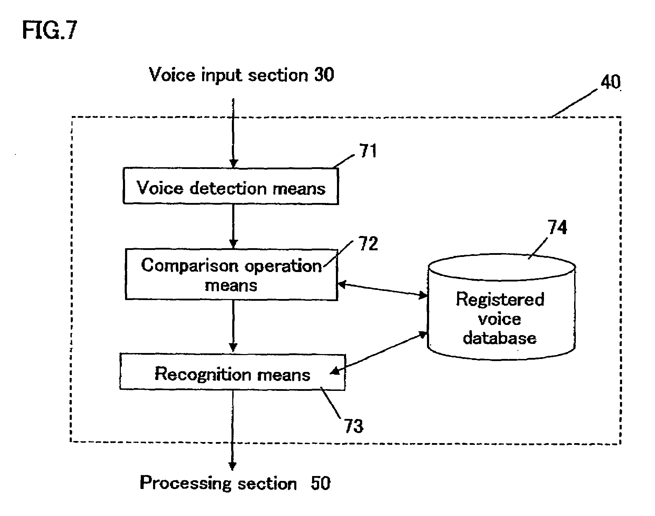

Figure **7** shows an exemplary internal structure of the voice recognition section **40.**

The voice input section **30** (microphone) converts voice into a voice signal waveform. The voice signal waveform is output to the voice recognition section **40.** The voice recognition section **40** includes voice detection means **71,** comparison operation means **72,** recognition means **73,** and a registered voice database **74.**

The voice detection means **71** cuts a part of the voice signal waveform input from the voice input section **30,** which satisfies a certain standard, as a voice interval actually produced by a user, and outputs the audio signal waveform in the interval to the comparison operation means **72** as a voice waveform. Herein, a certain standard for cutting out the voice interval may be, for example, that power of the signal waveform in a frequency band of 1kHz or less, which is generally a voice band of humans, is at a certain level or hither.

In the registered voice database **74**, voice waveforms of words which are expected to be uttered by the user and the words are registered with the correspondences therebetween.

The comparison operation means **72** sequentially compares voice waveforms input front the voice detection means **71** with the voice waveforms registered in the registered voice database **74.** The comparison operation means **72** calculates the degree of similarity for each of the voice waveforms registered in the registered voice database **74,** and outputs the calculated results to the recognition means **73.** Herein, a method for comparing two voice waveforms may be a method of comparing totals of differences in power components at respective frequencies after the voice waveform is subjected to frequency analysis such as Fourier transform or the like, or may be a method in which DP matching is performed with an expand and contract in time being taken into account in cepstrum feature quantity orMel cepstrum feature quantity which is further subjected to polar coordinate transformation after the frequency analysis. Moreover, for efficient comparison operation, the voice waveforms registered in the registered voice database **74** may be comparison factors used in the comparison operation means **72** (for example, power components of the respective frequencies). Further, among the voice waveforms registered in the registered voice database **74**, voice waveforms of voice produced unintentionally by the user, for example, cough, groan, and the like are registered, and, as the corresponding words, "unintentional voice production" is registered. Thus, it becomes possible to distinguish between the voice production intended by the user and the voice production which is not intended.

The recognition means **73** detects the voice waveform which has the highest degree of similarity from the degrees of similarities of the respective voice waveforms input from the comparison operation means **72.** The recognition means **73** decides the word corresponding to the voice waveform detected from the registered voice database **74** to convert the voice waveform into text, and output the text to the processing section **50.** When there is no significant difference among the similarities, it may determine that the input voice is noise and does not perform conversion from the voice waveform into the text. Alternatively, it may convert the voice waveform into the text such as "noise".

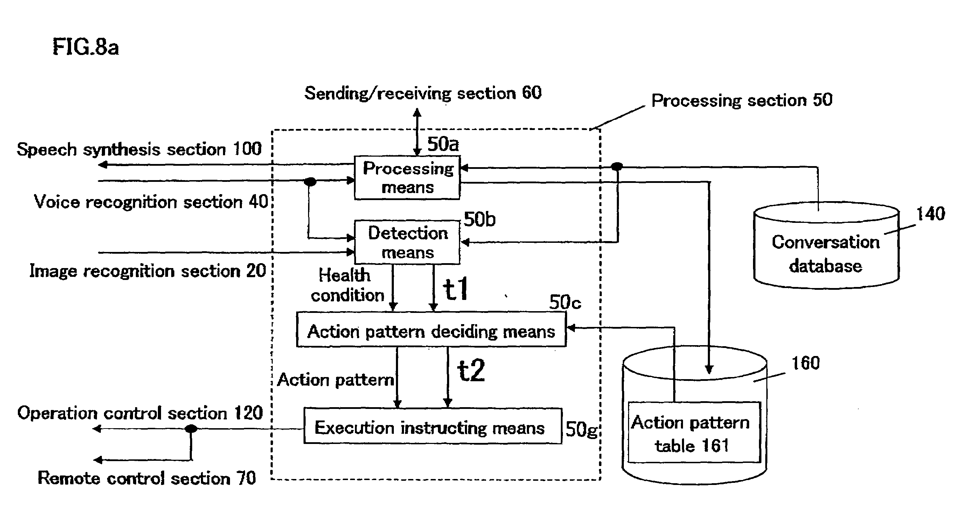

Figure **8a** shows an exemplary internal structure of the processing section **50** shown in Figure **2**.

The processing section **50** (processing means **50a**) searches a conversation database **140** based on the voice recognition results by the voice recognition section **40**, and generates a responding sentence. The responding sentence is output to a speech synthesis section **100.** The speech synthesis section **100** synthesizes the responding sentence into a speech. The synthesized speech is output from the audio output section **110** such as a speaker.

In the conversation database **140,** patterns of conversation and rules for generating responding sentences. The conversation database **140** further stores the relationships between the words (keywords) uttered by the user and health conditions of the user.

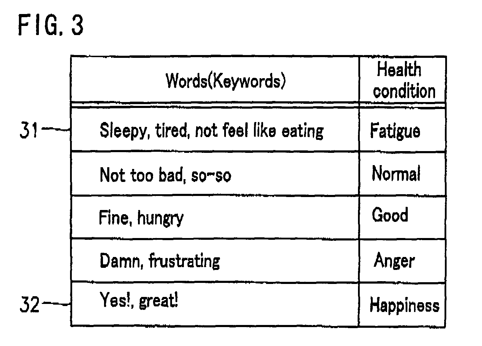

Figure **3** shows exemplary relationships between the keywords uttered by the user, which are stored in the conversation database **140,** and the health conditions of the user.

In the example shown in Figure **3**, the relationships between the keywords uttered by the user and the health conditions of the user are represented in a format of a table. For example, a row in this table indicates that keywords such as "sleepy", "tired", and "not feel like eating" correspond to the health condition (physical condition) of the user, "fatigue". A row **32** of the table shows that keywords such as "yes!" and "great!" correspond to the health condition (feeling) of the user, "pleasure".

The way to represent the relationships between the keywords uttered by the user and the health conditions of the user is not limited to that shown in Figure **3**. The relationships between the keywords uttered by the user and the health conditions of the user may be represented in any way.

The processing section **50** (detection means **50b**) extracts a keyword from the voice recognition result by the voice recognition section **40**, and searches the conversation database **140** using the keyword. Consequently, the processing section **50** (detection means **50b**) detects the health condition of the user from the keyword. For example, when the keyword extracted from the voice recognition result is one of "sleepy", "tired", and "not feel like eating", the processing section **50** (detection means **50b**) determines that the health condition of the user is "fatigue" with reference to the table as shown in Figure **3**.

Instead of or in addition to the above-described method using keywords, the health condition may be detected by detecting the level of the strength or deepness of the voice of the user based on the voice recognition result. For example, when the processing section **50** (detection means **50b**) detects that the level of the strength or deepness of the voice of the user equals to or lower than the predetermined level, the processing section **50** (detection means **50b**) determines that the health condition of the user is "fatigue".

Further, in addition to the voice recognition result by the voice recognition section **40,** the health condition of the user may be detected using the image recognition result by the image recognition section **20.** Alternatively, the health condition of the user may be detected by using only the image recognition result by the image recognition section **20.** For example, when the processing section **50** (detection means **50b**) detected that the user frequently blinks (or the user yawns), the processing section **50** (detection means **50b**) determines that the health condition of the user is "fatigue".

As such, the processing section **50** (detection means **50b**) may functions as detection means for detecting the health condition of the user based on the utterance of the user or the image recognition result.

An information database **160** stores information such as today' s weather and news, knowledge such as various common knowledge, information regarding the user (owner) of the robot **1** (for example, information such as sex, age, name, occupation, character, hobby, date of birth, and the like), information regarding the robot **1** (for example, information such as model number, internal structures and the like). The information such as today's weather and news is obtained by, for example, the robot **1** from outside via the sending/reoeiving section **60** (communication section) and the processing section **50,** and stored in the information database **160.** Further, the information database **160** stores the relationships between the health conditions of the user and action patterns as an action pattern table **161.**

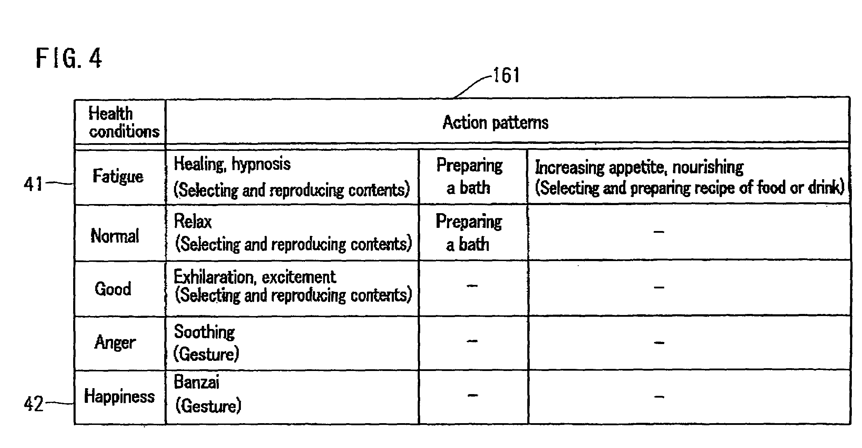

Figure **4** shows an exemplary action pattern table **161** stored in the information database **160.** The action pattern table **161** defines the relationships between the health condition of the user and the robot **1.**

In the example shown in Figure **4,** the health condition of the user and the action pattern of the robot **1** are represented in the format of a table. For example, a row **41** shows that the health condition of the user, "fatigue" corresponds to three kinds of action patterns of the robot **1**. Three kinds of action patterns are as follows.
1) Selecting and reproducing contents: Select contents (software) which produce a "healing" or "hypnotic" effect, and reproduce the selected contents (software) with a reproducing device
2) Preparing a bath: Prepare a bath in order to suggest the user to take a bath
3) Selecting and preparing a recipe of food or drink: Select a recipe of food or drink which "increases the appetite", and/or which is "nourishing", and prepare the food or drink following the selected recipe

A row **42** in the table shows that the health condition of the user, "pleasure", correspond to the action pattern of the robot **1**, "gesture of 'banzai' (raising arms for cheering)".

The way to represent the relationships between the health conditions of the user and the action patterns of the robot **1** is not limited to that shown in Figure **4**. The relationships between the health conditions of the user and the action patterns of the robot **1** may be represented in any way.

Examples of the action patterns of the robot **1** include: selecting the contents (software) which matches the health condition of the user and reproducing the selected contents (software) with a reproducing device; selecting a recipe of food or drink which matches the health condition of the user and preparing the food or drink following the selected recipe; preparing a bath; and telling joke for getting a laugh.

The processing section **50** (action pattern deciding means **50c**) searches the information database **160** (action pattern table **161**) using the health condition of the user detected by searching the conversation database **140** in response to a timing signal t1 output from the detection means **50b.** Consequently, the processing section **50** (the action pattern deciding means **50c**) determines the action pattern of the robot **1** in accordance with the health condition of the user. For example, when the health condition of the user is "fatigue", the processing section **50** (action pattern deciding means **50c**) determines one of the three action patterns defined in correspondence with "fatigue" as the action pattern of the robot **1** with reference to the table shown in Figure **4** (action pattern table **161**).

Herein, the processing section **50** (action pattern deciding means **50c**) can decide one of three action patterns as the action pattern of the robot **1** in various manner. For example, when priorities may be allocated to three action patterns, the action pattern of the robot **1** may be decided in descending order of priorities. The priorities may be varied depending on the time of the day. For example, the priority of "preparing a bath" may be made to be the highest during the time from 18:00 to 22:00, the priority of "selecting and preparing a recipe of food or drink" may be made to be the highest during 6:00 to 8:00, 11:00 to 13:00, and 17:00 to 19:00, and in other time, the priority of "selecting and reproducing contents" may be made to be the highest.

As described above, the processing section **50** (action pattern deciding means **50c**) functions as deciding means for deciding on the action pattern in accordance with the health condition of the user detected by the detection means **50b.**

The processing section **50** (execution instructing means **50g**) generates a control signal according to the decided action pattern in response to a timing signal t2 output from the action pattern deciding means **50c,** and outputs the control signal to an operation control section **120.**

The operation control section **120** drives various actuators **130** in accordance with a control signal output from the processing section **50** (execution instructing means **50g**). Thus, it becomes possible to operate the robot **1** in a desired manner.

For example, when the decided action pattern is the "gesture of 'banzai'", the operation control section **120** drives an actuator (a part of the actuator **130**) which moves "arms" of the robot **1** up and down in accordance with the control signal output from the processing section **50** (execution instructing means **50g**). When the decided action pattern is "selecting and reproducing contents", the operation control section **120** may drive an actuator (a part of the actuator **130**) for controlling "fingers of hands" of the robot **1** so as to hold a disc and set the held disc in a reproducing device in accordance with the control signal output from the processing section **50** (execution instructing means **50g**). For example, a plurality of discs are arranged and stored in a rack in a predetermined order.

As described above, the processing section **50** (execution instructing means **50g**) functions as execution instructing means for instructing execution of the action pattern decided by the action pattern deciding means **50c** to the operation control section **120.**

Alternatively, when the decided action pattern is "preparing a bath", the processing section **50** (execution instructing means **50g**) may control a remote control section **70** so as to send a remote control signal to a hot-water supply device. The hot-water supply device supplies an appropriate amount of hot-water of a desired temperature (or, supply an appropriate amount of water to a bath tab and then heat the water to the desired temperature) in accordance with a remote control signal. In this case, the processing section **50** (execution instructing means **50g**) functions as instruction indicating means for indicating the execution of the action pattern decided by the action pattern deciding means **50c** to the remote control section **70.**

Alternatively, when the decided action pattern is "selecting and reproducing contents", the processing section **50** (execution instructing means **50g**) may control a remote control section **70** so as to send a remote control signal to a reproducing device. The reproducing device selects the contents from discs set in the reproducing in accordance with the remote control signal for reproduction. If the reproducing device is connected to a disc changer which allows for a plurality of discs to be set, the reproducing device may select the contents from the plurality of discs in accordance with a remote control signal for reproduction. A list for selecting a musical piece including all the musical pieces in a plurality of discs may be stored in a memory in the processing section **50**. Alternatively, the reproducing device may read a list for selecting a musical piece of a disc from a header portion of the disc, and then store in a memory in the processing section **50** via the sending and receiving section **60.** In such a case, the processing section **50** (execution instructing means **50g**) functions as execution instructing means for instructing execution of the action pattern decided by the action pattern deciding means **50c** to the remote control section **70**.

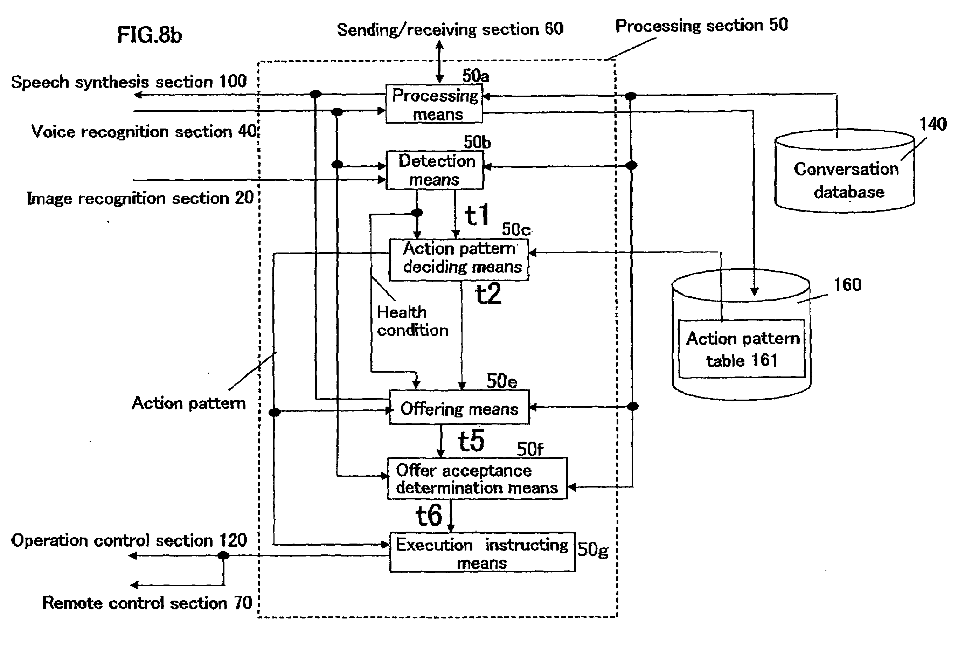

Figure **8b** shows another exemplary internal structure of the processing section **50** shown in Figure **2.** In the example shown in Figure **8b,** the processing section **50** (offering means **50e**) makes an offer of the decided action pattern to the user by a speech before it instructs execution of the action pattern. For example, when the decided action pattern is "preparing a bath", in response to the timing signal t2 output from the action pattern deciding means **50c,** the processing section **50** (offering means **50e**) may generate interrogative sentence (offering sentence) such as "You look tired. Shall I prepare a bath for you?" with reference to the conversation database **140,** and output to the speech synthesis section **100.** The speech synthesis section **100** synthesizes the interrogative sentence into a speech. The synthesized speech is output from the audio output section **110.**

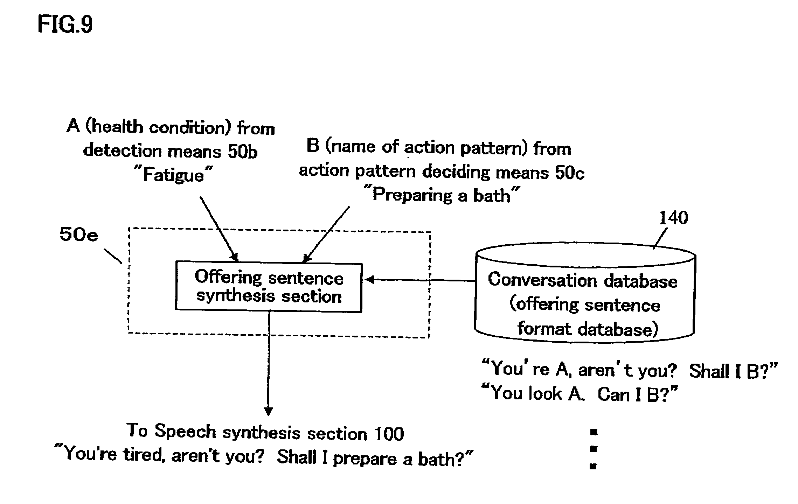

Next, how the offering means **50e** create offering sentences will be described with reference to Figure **9.** The offering means **50e** includes an offering sentence synthesis section therein. The conversation database **140** includes an offering sentence format database therein. In the offering sentence format database, a plurality of offering sentence formats corresponding to a plurality of offer expressions are recorded and stored. Herein, "offer expressions" are words and expressions which indicate a cause (A) which motivates the offer and a response (B) to the cause, such as, "You're A, aren't you? Shall I B7" or "You look A. Can I B?" as shown in Figure **9**, for example.

First, the offering means (offer synthesis section) **50e** selects an offering sentence format which matches the "detected health condition" from the offering sentence format database based on the "detected health condition" input from the detection means **50b** and the "decided action pattern" input from the action pattern deciding means **50c.** Next, the offering means (offer synthesis section) **50e** synthesizes an offering sentence by inserting the "detected health condition" into A in the offering sentence format, and the "decided action pattern" into B. For example, when the "detected health condition" is "fatigue", and the "decided action pattern" is "preparing a bath", the offering means (offer synthesis section) **50e** synthesizes an offering sentence, "You look tired. Shall I prepare a bath for you?". The offering sentence is output to the speech synthesis section **100.** The speech synthesis section **100** synthesizes the offering sentence into a speech. The synthesized speech is output from the audio output section **110.**

As described above, the processing section **50** (offering means **50e**) functions as offering means for making an offer of an action pattern decided by the action pattern deciding means **50c** to the user by a speech before it instructs the execution of the action pattern by using the conversation database (offering sentence format database) **140,** the speech synthesis section **100,** and the audio output section **110.**

The user gives an answer to the offer from the robot 1 whether to accept the offer or not. For example, the user gives an answer such as "yes", "yeah", "please do that" and the like as an indication to accept the offer (Yes). Alternatively, the user gives an answer such as "no", "no, thanks", "don't need that" and the like as an indication not to accept the offer (No). Such patterns of answers are previously stored in the conversation database **140**.

The processing section **50** (offer acceptance determination means **50f**) determines whether the answer of the user is an answer to accept the offer (Yes) or an answer not accept the offer (No) by analyzing the voice recognition result by the voice recognition section **40** with reference to the conversation database **140** in response to a timing signal t5 output from the offering means **50e.**

As described above, the processing section **50** (offer acceptance determination means **50f**) functions as offer acceptance determination means for determining whether the answer of the user is an answer to accept the offer (Yes) or an answer not accept the offer (No) by using the voice recognition section **40** and the conversation database **140.**

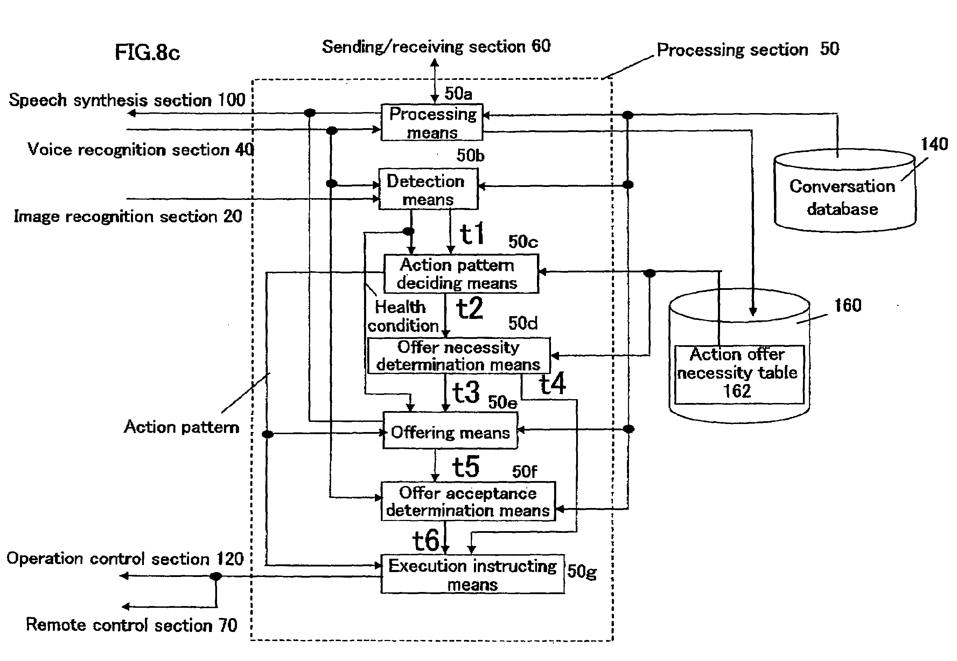

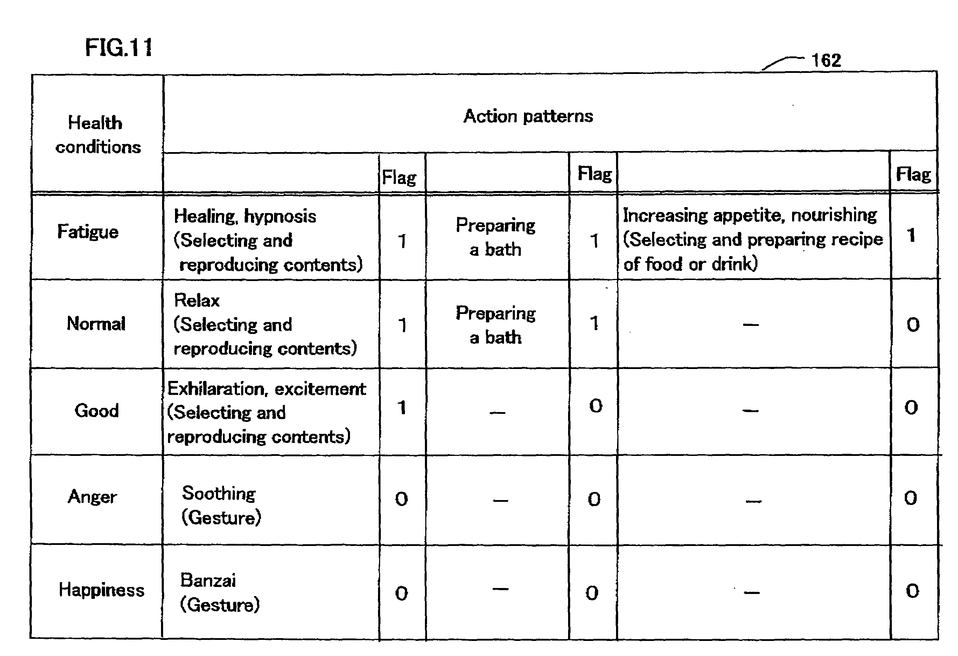

Figure **8c** shows another exemplary internal structure of the processing section **50** shown in Figure **2.** Whether it is necessary to make the offer of the decided action pattern to the user before execution of the action pattern may be determined. For example, by previously setting an action offer necessity table **162** shown in Figure **11** where flags indicating necessities of offers are previously allocated to the action patterns in the table shown in Figure **4,** the processing section **50** (offer necessity determination means **50d**) can determine whether the offer is necessary or not in accordance with values of the flags. For example, the processing section **50** (offer necessity determination means **50d**) makes an offer of an action pattern to the user when the value of the flag allocated to the action pattern is "1" before it instructs execution of the action pattern, and does not make an offer of an action pattern to the user when the value of the flag allocated to the action pattern is "0" before it instructs execution of the action pattern.

For example, regarding the action pattern of "preparing a bath", it is preferable that the offer to the user beforehand is required. Whether or not the user wants to take a bath or not largely depends on the mood at the time of the user. Thus, if the offer to the user beforehand is not required, it may be intrusive. For example, regarding the action pattern of the "gesture of 'banzai'", it is preferable that the offer to the user beforehand is not required. If the user is asked for permission every time the banzai gesture is performed, it may look foolish.

As described above, the processing section **50** (offer necessity determination means **50d**) functions as offer necessity determination means for determining whether or not it is necessary to make an offer of the decided action pattern to the user before it instructs execution of the action pattern by using the information database **160** (action offer necessity table **162**).

If the time of the day the action pattern is performed is always the same, or the action pattern is frequently performed, it is not desirable to make the offer of the action pattern to the user every time. On the other hand, regarding an action pattern which is not performed usually, it is preferable to confirm whether the user wants execution of the action pattern by making an offer of the action pattern to the user before execution of the action pattern is instructed.

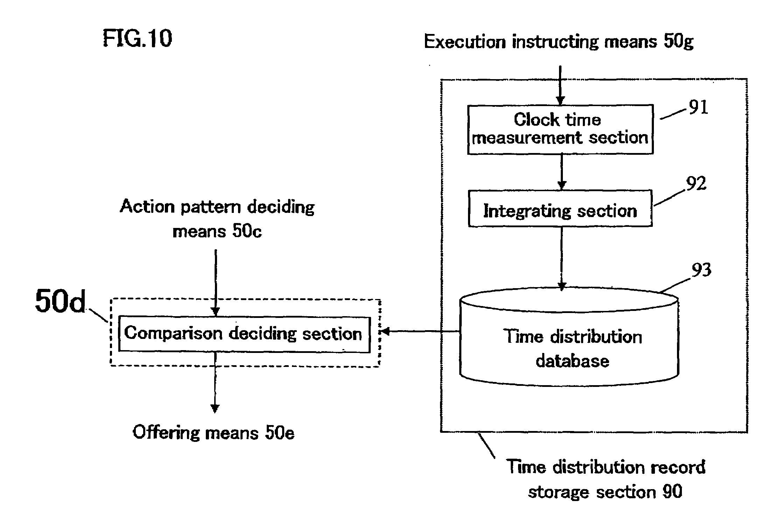

With reference to Figure **10,** the offer necessity determination means **50d** which implements the above-described function will be described. A time distribution record storage section **90** includes a clock time measurement section **91,** an integrating section **92,** and a time distribution database **93.** The offer necessity determination means **50d** includes comparison deciding section therein. The clock time measurement section **91** receives an input of the execution instructing means **50g**, measures the clock time when the action pattern is performed, and outputs to the integrating section **92.** The time distribution database **93** records and stores the number of times each of the action patterns is performed at every clock time. The integrating section **92** adds 1 to the number of times recorded in the time distribution database **93** at the measured clock time every time it receives input from the clock time measurement section **91**. The time distribution record storage section **90** accumulates history information of action patterns performed at every clock time as such. The offer necessity determination means (comparison deciding means) **50d** has pre-set values, and, when it receives an input from the action pattern deciding means **50c**, refers the number of times the action pattern is performed in the past at the clock time (or, in the time period) to the time distribution record storage section **90**, and compares with the pre-set value. The comparison deciding section determines that it is necessary to make offer of the action pattern when the number of times the action pattern is performed in the past is smaller than the pre-set value, and determines that it is not necessary to make an offer of the action pattern when the number of times the action pattern is performed in the past is larger than the pre-set value. The determined results is output from the offer necessity determination means **50d** as determination results of the offer necessity determination means **50d.**

As described above, the offer necessity determination means **50d** determines the necessity of making offer based on time distribution of the number of times the action pattern is performed.

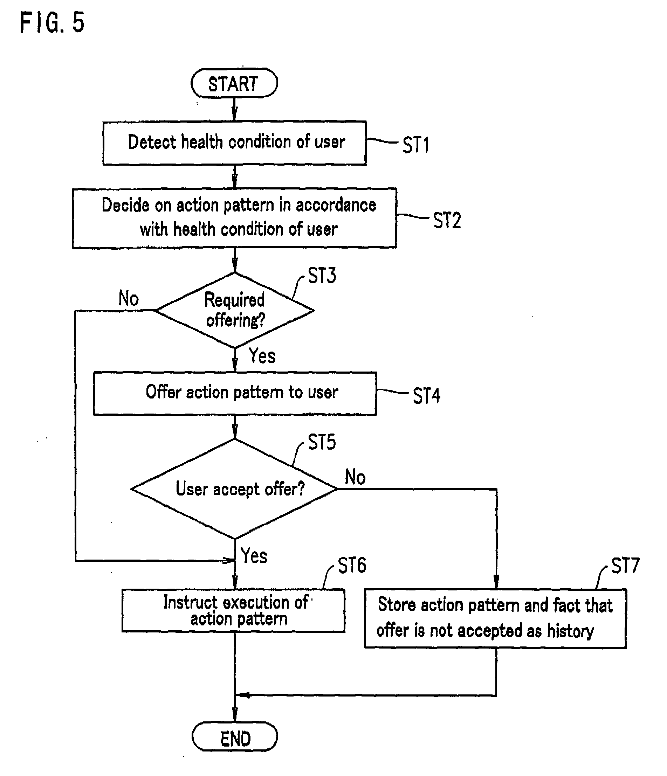

Figure **5** shows a procedure of process where the robot 1 detects the health condition of the user and instructs execution of an action pattern which matches the health condition of the user.

Step ST1: The health condition of the user is detected.

For example, the processing section **50** (detection means **50b**) extracts a keyword from the voice recognition result by the voice recognition section **40,** and searches the conversation database **140** using the keyword. As a result, the processing section **50** (detection means **50b**) can detect the health condition of the user from the keyword.

Hereinafter, an example of the conversation between the user and the robot **1** is shown. Herein, U denotes the utterance by the user, and S denotes to the speech of the robot **1.**
U: I'm tired today.
S: Looks like that.

As in this example, when the user utters keywords such as "sleepy", "tired", and "not feel like eating", the processing section **50** (detection moans **50b**) determines that the health condition of the user is "fatigue".

Step ST2: An action pattern is decided in accordance with the health condition of the user detected in step ST1.

For example, the processing section **50** (action pattern deciding means **50c**) searches the information database **160** (action pattern table **161**) using the health condition of the user. As a result, the processing section **50** (action pattern deciding means **50c**) can decide the action pattern corresponding to the health condition of the user. It is preferable that the action pattern is previously set as estimating the demand of the user.

Step ST3: Whether it is necessary to make an offer of the action pattern to the user before the instruction of execution of the action pattern decided in step ST2 is determined by the offer necessity determination means **50d**.

When the determined result in step ST3 is "Yes", the process goes to step ST4, and, when the determined result in step ST3 is "No", the process goes to step ST6.

Step ST4: The offer of the action pattern decided in step ST2 is given to the user by the offering means **50e** before the execution of the action pattern is instructed.

Hereinafter, an example of the conversation between the user and the robot **1** is shown. Herein, U denotes the utterance by the user, and S denotes to the speech of the robot **1**.
S: You look tired. Shall I reproduce contents (software) having a healing effect?
U: Yeah.

Step ST5: Whether or not the user give an answer to accept the action pattern offered by the robot **1** in step ST4 is determined by the offer acceptance determination means **50f.**

When the determined result in step ST5 is "Yes", the process goes to step ST6, and, when the determined result in step ST5 is "No", the process goes to step ST7.

Step ST6: Execution of the action pattern decided in step ST2 is instructed by the execution instructing means 50g.

Step ST7: The offered action pattern and the fact that the user did not accept (rejected) the offer are stored in the information database **160** as history information.

The history information is referred to from the next time to decide on contents of an action pattern in step ST2 from the next time. The priority allocated to the action pattern which is not accepted by the user can be made lower.

Instead of or in addition to step ST7, in the case where the offer is accepted by the user in step ST5, the offered action pattern and the fact that the user took up (accepted) the offer may be stored in the information database **160** as history information. The history information is referred to from the next time to decide on contents of an action pattern in step ST2. The priority allocated to the action pattern which is accepted by the user can be made higher.

As described above, it is preferable to vary the priorities allocated to action patterns in accordance with whether the offered action patterns are accepted by the user or not. This allows reflecting habits and the like of the user in deciding on the action patterns. As a result, it becomes possible to improve the percentage that the action pattern decided by the robot **1** actually matches the health condition of the user.

The user may make a counteroffer when the user did not accept the offer in step ST5. In such a case, the robot **1** receives the counteroffer and determines whether the counter offer is executable or not. When it is determined that the counteroffer is executable, the robot **1** updates the relationship between the health condition of the user and the action pattern of the robot **1** stored in the information database **160** (for example, updates the priorities of the action patterns in the table shown in Figure **4,** or, adds new patterns in the table shown in Figure **4**), and then instructs execution of the counteroffer. When it is determined that the counteroffer is not executable, the robot **1** notifies of the user that "the counteroffer cannot be performed". In this way, by providing the counteroffer from the user, habits and the like of the user can be reflected in deciding on the action patterns. As a result, it becomes possible to improve the percentage that the action pattern decided by the robot **1** actually matches the health condition of the user.

In Figure **5**, step ST3 may be omitted. In such a case, all the action patterns decided in accordance with the health conditions of the user are offered to the user before execution of the action patterns is instructed.

Further, in Figure **5**, steps ST3, ST4, ST5, and ST7 may be omitted. In such a case, all the action patterns decided in accordance with the health condition of the user are instructed to be performed immediately without waiting for an answer from the user.

As described above, according to the present embodiment, the health condition of the user is detected, and the action pattern in accordance with the health condition of the user is decided. Thus, the user can be relieved from a burden of wearing various sensors. Furthermore, the user feels that the robot is an entity that cares about the health condition of the user (good friend).

Further, a system to make an offer of the action pattern to the user before indicating execution of the action pattern may be employed. In such a case, the user has a final decision on whether to accept the offer or not. Thus, the user is not force by the robot to accept the offer, and has a high degree of freedom in judgment. This allows suppressing runaway of the robot, and also for the user to feel familiar to the robot as a user-friendly entity.

According to a survey conducted by JMA Research Institute Inc., the most poplar dream robot imagined by consumers was a "robot pet more like a real pet". Robots of coexistent or entertainment type closely related humans' lives which share a living space with humans are expected.

It could be understood that the robot as an example of the interactive apparatus according to the present invention is a friendly and useful robot closely related to humans' lives. Such a robot can help the life of the user and may be a good friend of the user.

The contents (software) to be reproduced by the reproducing device may include at least one of video data, audio data, and lighting control data. It is possible to reproduce audio data recorded on a recording medium (such as DVD) in synchronization with reproduction of video data recorded in the recording medium. It is also possible to reproduce lighting control data recorded on a recording medium (such as DVD) in synchronization with reproduction of audio data and/or video data. Such a synchronized reproduction allows to realize contents (software) having a significant "healing" effect and/or "hypnotic" effect.

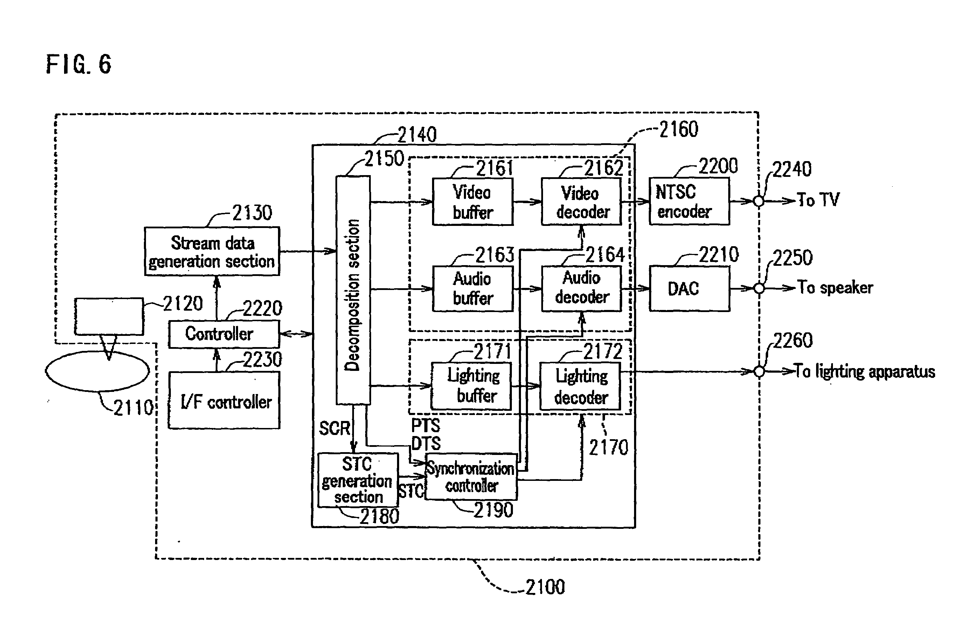

Figure **6** shows an exemplary structure of a reproducing apparatus **2100** which allows synchronized reproduction of the audio data and/or video data, and the lighting control data. The reproducing apparatus **2100** is connected to an audio outputting device (for example, a speaker) and a video outputting device (for example, a TV). Thus , the reproducing apparatus **2100** can change a lighting pattern of a lighting apparatus (for example, at least one of light intensity and color of light of the lighting apparatus) in conjunction with music and/or video provided by a recording medium.

The reproducing apparatus **2100** includes a controller 2220, an interface controller (I/F controller) **2230,** and a reading out section **2120.**

The controller **2220** controls the entire operation of the reproducing apparatus **2100** based on an operation command from the user which is to be input into the I/F controller **2230** or a control signal provided from a decoding section **2140**.

The I/F controller **2230** detects an operation by the user (for example, a remote control signal from the remote control section **70**(Figure **2**)), and outputs an operation command corresponding to the operation (for example, a reproduction command) to the controller **2220.**

The reading out section **2120** reads out information recorded on a recording medium **2110.**

The recording medium **2110** is, typically, a DVD (Digital Versatile Disk). However, the recording medium 2110 is not limited to DVD. The recording medium **2110** may be any type of recording medium. In the following description, an example in which the recording medium **2110** is a DVD will be described. In this case, the reading out section **2120** is, for example, an optical pickup.

As a format for the data recorded in the recording medium **2110,** a modified version of a format in conformity with DVD-Image standard is used. Specifically, a format with a lighting pack (L_PCK) newly provided in VOBU is used. Data of L_PCK is data for outputting lighting control data in synchronization with presentation data.

MPEG-2 (Moving Picture Experts Group 2) defines two types of schemes as a scheme for multiplexing any number of encoded streams and reproducing the streams in synchronization in order to be compatible with a wide range of applications. The two types of schemes are a program stream (PS) scheme and a transport stream (TS) scheme. Digital storage media such as DVD employs the program stream (PS) scheme. In the following description, the program stream (PS) scheme defined by MPEG-2 is abbreviated as "MPEG-PS scheme", and the transport stream (TS) scheme defined by MPEG-2 is abbreviated as "MPEG-TS scheme".

Each of NV_PCK, A_PCK, V_PCK, and SP_PCK employs a format in conformity with the MPEG-PS scheme. Thus, L_PCK also employs a format in conformity with the MPEG-PS scheme.

The reproducing apparatus **2100** further includes a stream data generation section **2130,** and the decoding section **2140.**

The stream data generation section **2130** generates stream data including encoded AV data and encoded lighting control data based on the output from the reading out section **2120.** Herein, "encoded AV data" refers to data including at least one of encoded audio data and encoded video data.

The stream data generated by the stream data generation section **2130** has a format in conformity with the MPEG-PS scheme. Such a stream data can be obtained by, for example, receiving information recorded in the DVD **2120** in the form of an RF signal, digitizing and amplifying the RF signal, and performing EFM and demodulation process. The structure of the stream data generation section **2130** may be same as the one known. Thus, detailed description is omitted here.

The decoding section **2140** includes a decomposition section **2150,** an AV data decoding section **2160,** a lighting control data decoding section **2170,** an STC generation section **2180,** and a synchronization controller (control section) **2190.**

The decomposition section **2150** receives stream data having a format in conformity with the MPEG-PS scheme from the stream data generation section **2130,** and decomposes the stream data into encoded AV data and encoded lighting control data. Such decomposition is performed with reference to an identification code in a PES packet header (stream_id). The decomposition section **2150** is, for example, a demultiplexer.

The AV data decoding section **2160** outputs AV data by decoding the encoded AV data. Herein, "AV data" refers to data including at least one of audio data and video data.

The AV data decoding section **2160** includes: a video buffer **2161** for temporarily storing encoded video data which is output from the decomposition section **2150;** a video decoder 2162 for outputting video data by decoding the encoded video data; an audio buffer **2163** for temporarily storing encoded audio data which is output from the decomposition section **2150;** and an audio decoder **2164** for outputting the audio data by decoding the encoded audio data.

The lighting control data decoding section **2170** outputs the lighting control data by decoding the encoded lighting control data. Herein, "lighting control data" is data for controlling a plurality of pixels included in the lighting apparatus.

The lighting control data decoding section **2170** includes: a lighting control buffer **2171** for temporarily storing the encoded lighting data which is output from the decomposition section **2150;** and a lighting decoder **2172** for outputting the lighting control data by decoding the encoded lighting control data.

The STC generation section **2180** generates STC (System Time Clock). STC is obtained by adjusting (increasing or decreasing) a frequency of a reference clock of 27MHz based on SCR. STC is a reference time used for encoding data which is reproduced when the encoded data is decoded.

The synchronization controller **2190** controls the AV data decoding section **2160** and the lighting control data decoding section **2170** such that the timing for the AV data decoding section **2160** to output AV data and the timing for the lighting control data decoding section **2170** to output the lighting control data are in synchronization.

Controlling such a synchronized reproduction is achieved by, for example, controlling the video decoder **2162** such that an access unit of video data is output from the video decoder **2162** when STC and PTS match, controlling the audio decoder **2164** such that an access unit of video data is output from the audio decoder **2164** when STC and PTS match, and controlling the lighting decoder **2172** such that an access unit of video data is output from the lighting decoder **2172** when STC and PTS match.

The synchronization controller **2190** may control the AV data decoding section **2160** and the lighting control data decoding section **2170** such that the timing for the AV data decoding section **2160** to decode AV data and the timing for the lighting control data decoding section **2170** to decode the lighting control data are in synchronization.

Controlling such a synchronized reproduction is achieved by, for example, controlling the video decoder **2162** such that an access unit of video data is decoded by the video decoder **2162** when STC and DTS match, controlling the audio decoder **2164** such that an access unit of video data is decoded by the audio decoder **2164** when STC and DTS match, and controlling the lighting decoder **2172** such that an access unit of video data is decoded by the lighting decoder **2172** when STC and DTS match.

As described above, in addition to controlling the timing to output access units of video data, audio data, and lighting control data, or, instead of controlling the timing to output access units of video data, audio data, and lighting control data, controlling the timing to decode access units of video data, audio data, and lighting control data may be performed. This is because, sometimes, the timing (order) to output the access units and the timing to decode the access unit are different from each other. Such a control enables synchronized reproduction of video data, audio data, and lighting control data.

The video data output from the video decoder **2162** is output to an external device (for example, TV) via an NTSC encoder **2200.** The video decoder **2162** and the TV may be directly connected to each other via an output terminal **2240** of the reproducing apparatus **2100,** or may be indirectly connected via a home LAN.

The audio data output from the audio decoder **2164** is output to an external device (for example, speaker) via a digital to analog converter (DAC) **2210.** The audio decoder **2164** and the speaker may be directly connected via an output terminal of the reproducing apparatus **2100,** or may be indirectly connected via a home LAN.

The lighting control data output from the lighting decoder **2172** is output to an external device (for example, lighting apparatus). The lighting decoder **2172** and the lighting apparatus may be directly connected via an output terminal **2260** of the reproducing apparatus **2100,** or may be indirectly connected via a home LAN.

The stream data generated by the stream data generation section **2130** may include encoded sub-video data, or may include navigation data. For example, when the stream data include the encoded sub-video data and the navigation data, the decomposition section **2150** decomposes the stream data into the encoded sub-video data and navigation data. Although not shown in Figure **6,** the decoding section **2140** may further includes a navipaok circuit, a sub-picture decoder, and a closed caption data decoder. The navipack circuit generates a control signal by processing the navigation data, and outputs the control signal to the controller **2220.** The sub-picture decoder decodes the encoded sub-video data and outputs the sub-video data to the NTSC encoder **2200.** The closed caption data decoder decodes the encoded closed caption data included in the encoded video data and outputs the closed caption data to the NTSC encoder **2200.** Since the functions of these circuits are known and are not related to the subject matter of the present invention, the detailed description thereof is omitted. As described above, decoding section **2140** may include a known structure which is not shown in Figure **6.**

As shown in the above description, according to the reproducing apparatus **2100** shown in Figure **6,** a reproducing apparatus which allows that the lighting control data recorded on a recording medium is reproduced in synchronization with reproduction of the audio data and/or video data recorded on the recording medium. By connecting the audio outputting device (for example, speaker), the video outputting device (for example, TV), and the lighting apparatus to the reproducing apparatus, it becomes possible to change lighting pattern in conjunction with music and/or video provided by the recording medium. Examples of the lighting patterns having a "healing" effect include a lighting pattern representing sunlight passing between tree branches.

### INDUSTRIAL APPLICABILITY

As described above, according to interactive apparatus of the present invention, the health condition of the user is detected, and the action pattern in accordance with the health condition of the user is decided. Thus, the user can be relieved from a burden of wearing various sensors. Furthermore, the user feels that the interactive apparatus is an entity that cares about the health condition of the user (good friend). As a result, the value of the interactive apparatus is increased, and satisfaction and a desire for possession of the user toward the interactive apparatus are increased.

## Claims

1. An interactive apparatus, comprising:
detection means for detecting a health condition of a user;
deciding means for deciding on an action pattern in accordance with the health condition of the user detected by the detection means;
execution instructing means for instructing execution of the action pattern decided by the deciding means;
offering means for making an offer of the action pattern to the user with a speech before instructing execution of the action pattern decided by the deciding means; and
determination means for determining whether an answer of the user to the offered action pattern is an answer to accept the offered action pattern or not,
wherein the execution instructing means instructs execution of the offered action pattern when the answer of the user is determined to be the answer to accept the offered action pattern.

2. An interactive apparatus according to claim 1, wherein the detection means detects the health condition of the user based on utterance of the user.

3. An interactive apparatus according to claim 2, wherein the detection means detects the health condition of the user based on keywords uttered by the user.

4. An interactive apparatus according to claim 1, further comprising offer necessity determination means for determining whether it is required to make an offer of the action pattern to the user before instructing execution of the action pattern decided by the deciding means,
wherein the offering means makes an offer of the action pattern to the user with a speech when it is determined that making an offer of the action pattern to the user is required before instructing execution of the action pattern.

5. An interactive apparatus according to claim 4, wherein the offer necessity determination means determines necessity of making an offer in accordance with a value of a flag indicating a necessity of making an offer which is previously allocated to the action pattern.

6. An interactive apparatus according to claim 4, wherein the offer necessity determination means determines necessity of making an offer based on time distribution of the number of times the action pattern is performed.

7. An interactive apparatus according to claim 1, wherein the deciding means decides one of a plurality of action patterns to which priorities are respectively allocated as an action pattern in accordance with the health condition of the user, and changes the priority allocated to the action pattern in accordance with whether or not the action pattern is accepted by the user.

8. An interactive apparatus according to claim 1, further comprising storage means for storing the action pattern in accordance with the health condition of the user,
wherein the deciding means decides on the action pattern by using the action pattern stored in the storage means.

9. An interactive apparatus according to claim 1, wherein the action pattern offered by the offering means to the user includes selecting contents to be reproduced by a reproducing device.

10. An interactive apparatus according to claim 9, wherein the contents include audio data, video data, and lighting control data, and the reproducing device changes at least one of light intensity and color of light of a lighting apparatus based on the lighting control data.

11. An interactive apparatus according to claim 1, wherein the interactive device has at least one of an agent function and a traveling function.

12. An interactive apparatus according to claim 1, wherein the health condition of the user represents at least one of feelings of the user and a physical condition of the user.

13. An interactive apparatus, comprising:
a voice input section for converting a voice produced by the user into a voice signal,
a voice recognition section for recognizing words uttered by the user based on the voice signal output from the voice input section;
a conversation database in which words expected to be uttered by the user are previously registered, and which stores correspondences between the registered words and the health condition of the user;
detection means for detecting the health condition of the user by checking the words recognized by the voice recognition section against the words registered in the conversation database, and deciding on the health condition of the user in accordance with the checking result;
deciding means for deciding on an action pattern in accordance with the health condition of the user detected by the detection means based on an action pattern table storing correspondences between the health condition of the user and action patterns of the interactive apparatus;
execution instructing means for instructing execution of the action pattern decided by the deciding means;
offering means for synthesizing an offering sentence based on an output result of the detection means and an output result of the deciding means and making an offer of the action pattern to the user with a speech before instructing execution of the action pattern decided by the deciding means; and
determination means for determining whether an answer of the user to the offered action pattern is an answer to accept the offered action pattern or not,
wherein the execution instructing means instructs execution of the offered action pattern when the answer of the user is determined to be the answer to accept the offered action pattern.

14. An interactive apparatus according to claim 13, further comprising:
means for receiving an action pattern which is counter-offered by the user with respect to the offered action pattern;
means for the interactive apparatus to determine whether the counter-offered action pattern is executable or not; and
means for updating the correspondences between the health condition of the user and the action patterns of the interactive apparatus which are stored in the action pattern table when the interactive apparatus determines that the counter-offered action pattern is executable.
